# EUROPEAN PATENT SPECIFICATION

(11) **EP 2 653 141 B1**
(45) Date of publication and mention of the grant of the patent: **23.11.2016**
(21) Application number: 13152155.1
(22) Date of filing: 22.01.2013
(51) Int. Cl.: A61F 13/471, A61F 5/453

(54) **Male diaper for urinary protection**
Windel für Männer zum Harnschutz
Couche de protection urinaire masculine

(30) Priority: 17.04.2012 ES 201230410 U
(43) Date of publication of application: 23.10.2013
(73) Proprietor: Gutierrez Fernandez, Isabel Aurora, 33418 Santiago de Ambiedes-Gozon (Asturias) (ES); Bernardo Gutierrez, Rafael, 33418 Santiago de Ambiedes-Gozon (Asturias) (ES)
(72) Inventor: Gutierrez Fernandez, Isabel Aurora, 33418 SANTIAGO DE AMBIEDES-GOZON (Asturias) (ES)
(74) Representative: Ungria López, Javier

(56) References cited:
- WO-A1-2010/134286
- JP-A- 2006 141 872

## Description

### OBJECT OF THE INVENTION

The present invention, such as it is expressed in the statement of this specification, relates to a male diaper for urinary protection that is intended to be arranged around the penis being fixed in correspondence with the base of the male organ, such that the diaper of the invention has the function of absorbing and retaining the urine of the sick user, whose body does not control the urination of such urine, being essentially intended for people suffering long-term illness that are forced to be bedridden and/or in a wheelchair.

The application of the diaper of the invention is even more practical and beneficial when applied to patients, whose body cannot control the anal sphincter either, such that the diaper will prevent the mixing of the urine and the faeces, therefore avoiding possible infections of the male organ and adjacent areas.

### BACKGROUND OF THE INVENTION

In the present day, men and women who do not control urination use a diaper with a known structure that is fixed to the waist of the user as any known diaper.

However, sick people who have difficulties in controlling urination and the expulsion of faeces, have the additional problem that both elements are mixed, generating a greater problem which consists of the possibility that an infection may appear in the area that is in contact with the mixture of both elements.

Document WO 2010/134286 A1 describes an absorbent product for male comprising a bag-like main body part having an opening at an upper end, and two hydrophilic internal sheets which are band-like members located in the main body part. An upper end part of each internal sheet is fixed on an upper part of the main body part and the other portions are not bonded to the main body part. Internal sheet elastic members each extending in an up-down direction are bonded to each internal sheet, and by contraction of these members, gathers are formed in the internal sheet. In the absorbent product, since urine moves in the internal sheet comparatively slowly through the gathers formed in the internal sheet, a time required for the urine to reach the bottom part of the bag part can be increased. As the result, a large area of absorbent part can be utilized to absorb the urine effectively.

Document JP2006141872A describes an urine collecting bag that comprises a bag-like body with a front face part and a rear face part. The body also comprises a rear opening part formed in the rear face part to insert the male genital organs; an absorber capable of absorbing and holding urine and a front opening part formed in the front face part of the body to pull out the tip part of the male genital organs inserted in the body. The front face part has an upper front face part and a lower front face part with its upper end part overlapping with the lower end part of the upper front face part, and the front opening part is formed between the upper front face part and the lower front face part.

### DESCRIPTION OF THE INVENTION

With the purpose of achieving the objectives and avoiding the drawbacks mentioned in the preceding paragraphs, the invention proposes a male diaper for urinary protection that integrates in principle multiple layers of different materials: at least one waterproof inner layer and at least one outer layer for absorbing the urine of a man who does not control the urination of such urine.

It is characterized in that it comprises a bag-shaped surrounding body, within which the group of penis and genitals of a man is housed, such surrounding body having a mouth adjustable to the starting junction of the group of penis and genitals.

The structure that delimits the mouth of the surrounding body integrates an adjusting device, in the application position of which it generates an adjusted adaptation of such mouth against the starting junction of the group of penis and genitals of the man.

In an embodiment of the invention, the adjusting device comprises self-adhesive strips arranged on both sides of at least one open cut interrupting the continuity of the structure of the material delimiting the mouth of the surrounding body.

Next, in order to facilitate a better understanding of this specification and forming an integral part of the same some figures are accompanied wherein with illustrative character and without limitation the object of the invention has been represented.

### BRIEF DESCRIPTION OF THE DRAWINGS

**Figure 1****.-** It shows a perspective view of the male diaper for urinary protection, object of the invention, according to a first embodiment.
**Figure 2****.-** It shows a second embodiment of the diaper of the invention.

### DESCRIPTION OF AN EXAMPLE OF EMBODIMENT OF THE INVENTION

Considering the numbering adopted in the figures, the male diaper for urinary protection contemplates the following nomenclature used in the description:
1.- Surrounding body
2.- Surrounding body
3.- Mouth
3'.- Folds
4.- Mouth
5.- Elastic annular element
6.- Open cut
7.- Self-adhesive strips.

It comprises a surrounding body (1, 2) with a bag-shaped structure, which integrates an adjustable mouth (3, 4) to be able to adapt to the contour of the starting junction of the group of penis and genitals of a man, all of this with the purpose of ensuring the immobility of the diaper by its mouth (3, 4) around such starting junction, and the penis and the genitals are housed inside the surrounding structure of the diaper comfortably.

In this way on one hand prevention of the mixing of the urine of the patient with the faeces is achieved, and on the other hand, the urine absorption and retention in its entirety is achieved by the inner structure of the diaper which is known, without the moisture of the diaper being transmitted outside. This is achieved also through the outer structure of the aforementioned diaper, which is also known.

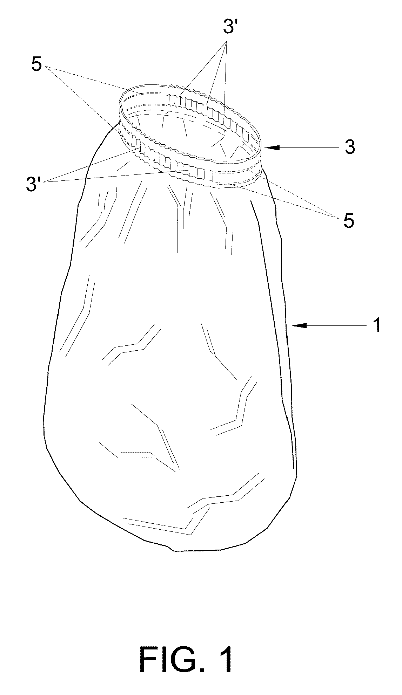

In an embodiment of a diaper, as it is shown in figure 1, the mouth (3) of the diaper integrates an elastic annular element (5) purpose of which is to close the mouth of the diaper, such that in resting position the mouth (3) is delimited by a narrowing, whereby when putting the diaper, it is necessary to first enlarge such mouth (3) manually during the placement process, against the resistance of the elastic annular element (5) until placing said mouth (3) at the height of the starting junction of the group of penis and genitals of the user, for subsequently slowly release such mouth (3) until the elasticity of the same fits and adjusts against the contour of such starting junction.

In this embodiment, the mouth (3) of the diaper includes a succession of folds (3') caused by the traction generated by the elasticity of the elastic annular element (5) integrated inside the internal structure of the diaper.

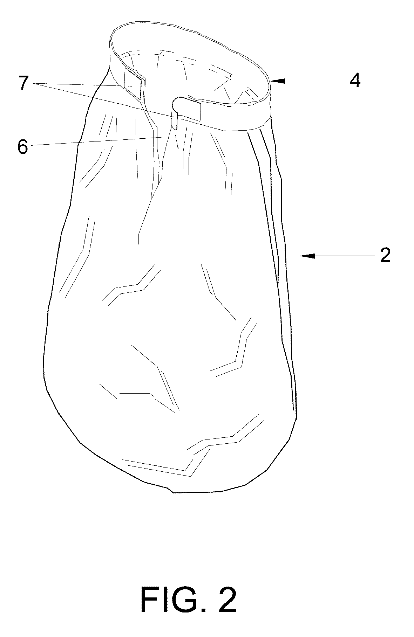

In an embodiment of the invention shown in figure 2, in correspondence with the mouth (4) of the diaper, the surrounding structure of the diaper incorporates an open cut (6), such that in this case, the areas close to such open cut (6) incorporate self-adhesive strips (7) that overlap and are joined together when such mouth (4) narrows when the same is adapted to the contour of the starting junction of the group of penis and genitals of the user at the moment of placing the diaper.

The area delimiting the material of the mouth (4) of the diaper of the invention is about 40 cm. although other larger or smaller sizes are not excluded.

## Claims

1. Male diaper for urinary protection, integrating multiple layers of different materials: at least one waterproof outer layer and at least one inner layer for absorbing the urine of a man who does not control urination of such urine; the male diaper comprising a bag-shaped surrounding body (1, 2), within which the group of penis and genitals of a man is housed; the surrounding body (1, 2) having a mouth (3, 4) adjustable to the starting junction of the group of penis and genitals; and the structure delimiting the mouth (3, 4) of the surrounding body (1, 2) integrating an adjusting device, such that in the application position of the surrounding body (1, 2), such adjusting device generates an adjusted adaptation of such mouth (3, 4) against the starting junction of the group of penis and genitals, the male diaper further comprising at least one open cut (6) interrupting the continuity of the structure of the material delimiting the mouth (4) of the surrounding body (2), **characterized in that** the adjusting device of the mouth (4) comprises self-adhesive strips (7) arranged on both sides of the at least one open cut (6).

## Patentansprüche

1. Windel für Männer zum Harnschutz, die mehrere Schichten aus unterschiedlichen Materialien aufweist: mindestens eine wasserdichte Außenschicht und mindestens eine Innenschicht zur Aufnahme des Urins eines Mannes, der die Abgabe des Urins nicht kontrolliert; wobei die Windel für Männer einen beutelförmigen umschließenden Körper (1, 2) aufweist, in welchem der Penis und die Geschlechtsteile eines Mannes als Gruppe aufgenommen werden; wobei der umschließende Körper (1, 2) eine Öffnung (3, 4) hat, die auf den Beginn des Übergangs der Gruppe aus Penis und Genitalien einstellbar ist; und wobei die Struktur, die die Öffnung (3, 4) des umschließenden Körpers (1, 2) begrenzt, eine Einstelleinrichtung enthält derart, dass in der Gebrauchsstellung des umschließenden Körpers (1, 2) die Einstelleinrichtung eine eingestellte Anpassung der Öffnung (3, 4) an den Beginn des Übergangs der Gruppe aus Penis und Genitalien erzeugt, wobei die Windel für Männer ferner mindestens einen Öffnungseinschnitt (6) aufweist, der die zusammenhängende Struktur des Materials unterbricht, das die Öffnung (4) des umschließenden Körpers (2) begrenzt, **dadurch gekennzeichnet, dass** die Einstelleinrichtung der Öffnung (4) selbsthaftende Streifen (7) aufweist, die auf beiden Seiten des mindestens einen Öffnungseinschnitts (6) angeordnet sind.

## Revendications

1. Couche de protection urinaire masculine, comprenant plusieurs couches de différents matériaux : au moins une couche externe imperméable et au moins une couche interne pour absorber l'urine d'un homme qui ne contrôle pas la miction d'une telle urine ; la protection urinaire masculine comprenant un corps périphérique en forme de sachet (1, 2) à l'intérieur duquel est logé le groupe composé du pénis et des testicules d'un homme ; le corps périphérique (1, 2) ayant une ouverture (3, 4) ajustable sur la jonction de départ du groupe composé du pénis et des testicules ; et la structure délimitant l'ouverture (3, 4) du corps périphérique (1, 2) comprenant un dispositif d'ajustement, de sorte que dans la position d'application du corps périphérique (1, 2), un tel dispositif d'ajustement génère une adaptation ajustée d'une telle ouverture (3, 4) contre la jonction de départ du groupe composé du pénis et des testicules, la protection urinaire masculine comprenant en outre au moins une découpe ouverte (6) interrompant la continuité de la structure du matériau délimitant l'ouverture (4) du corps périphérique (2), **caractérisé en ce que** le dispositif d'ajustement de l'ouverture (4) comprend des bandes autoadhésives (7) agencées des deux côtés de la au moins une découpe ouverte (6).
